Europäisches Patentamt

European Patent Office

Office européen des brevets

(11) Numéro de publication: **0 346 552**
**A1**

(12) **DEMANDE DE BREVET EUROPEEN**

(21) Numéro de dépôt: 88420199.7

(22) Date de dépôt: **15.06.88**

(51) Int. Cl.⁴: **C07C 179/06 , C07C 178/00**

(43) Date de publication de la demande:
**20.12.89 Bulletin 89/51**

(84) Etats contractants désignés:
**BE DE ES GB IT**

(71) Demandeur: **Leneueu-Delanef, Marie-Laure**
**61, rue Dedieu**
**F-69100 Villeurbanne(FR)**

(72) Inventeur: **Jacquier, René**
**32, rue Louis Thevenet**
**F-69004 Lyon(FR)**
Inventeur: **Guichard, Pierre**
**Les Grandes Terres**
**F-69380 Dommartin(FR)**

(54) **Nouveau procédé pour la production de dérivés organiques oxoniums et plus particulièrement de peroxydes de terpène oxoniums.**

(57) Procédé pour la production de péroxydes de terpènes oxoniums, ce en quantité massive, immédiatement disponible et à un niveau optimal de pureté.

L'invention permettant d'obtenir les produits (péroxydes de terpène oxoniums) et les appications ci-dessous décrites, consiste à envoyer un courant gazeux chargé de vapeurs d'essence de térébenthine vers un quelconque dispositif placé dans l'environnement proche de l'arc électrique, et, ou du générateur de rayons ultra-violets, pour refroidir ce dispositif, initialement porté à une température comprise entre 55°C et 2.950°C, jusqu'à une température comprise entre 35°C et 280°C.

Les dispositifs d'un tel procédé, selon l'invention, peuvent être un arc électrique (4), des électrodes (3), un tube (1), (5), (10), une résistance électrique (9), un courant d'air chaud (13) et en général tout autre(s) chose(s), pouvant être initialement chauffée(s), puis refroidite(s) par un courant gazeux chargé de vapeur d'essences de térébenthine.

Le procédé, selon l'invention, a trait aux produits obtenus, en l'occurence des péroxydes de terpène oxonium, et à leur applications médicales particulièrement en inhalations, notamment dans les domaines relevant de l'amélioration de la santé humaine et animale, de la prévention et du traitement des maladies, du maintien et de la mise en forme, de la désinfection, de la désodorisation et de la création d'ambiances.

FIG. 1

## NOUVEAU PROCEDE POUR LA PRODUCTION DE DERIVES ORGANIQUES OXONIUMS, ET PLUS PARTI-CULIEREMENT DE PEROXYDES DE TERPENE OXONIUMS

La présente invention a pour objet un nouveau procédé pour la production de dérivés organiques oxoniums et, plus particulièrement de péroxydes de terpène oxoniums ce, en quantité massive, immédiatement disponible et à un niveau optimal de pureté.

Elle a trait aux produits obtenus et à leurs applications médicales particulièrement en inhalations, notamment dans les domaines relevant de l'amélioration de la santé humaine et animale, de la prévention et du traitement des maladies, du maintien et de la mise en forme, de la désinfection, de la désodorisation et de la création d'ambiances.

L'on sait depuis longtemps produire des dérivés organiques oxoniums et plus particulièrement des péroxydes de terpène oxoniums ce, notamment, selon les procédés décrits dans les brevets de Monsieur René JACQUIER :
- Patent spécification 651.965
- Date de l'application et classification de la spécification complète publiée le 2 janvier 1947
- Spécification complète publiée le 11 avril 1951 sous cette application qui a été faite à l'origine dans les sections 31 de l'acte des brevets et dessins, 1907 à 1946. La spécification a été ouverte à l'examen public le 8 juillet 1947.

Mais l'utilisation de ces procédés soulèvent de grandes difficultés, ainsi :
- Un premier procédé consiste à envoyer un courant gazeux chargé de vapeurs d'essence de térébenthine sur la flamme d'un gaz, en l'occurence l'hydrogène, pour refroidir cette flamme jusqu'à une température comprise entre 45°C et 150°C.

Ce procédé donne des résultats satisfaisants quand aux produits obtenus et à leurs applications médicales mais nécessite l'emploi de l'hydrogène avec pour inconvéniants principaux :
* Le coût du conténeur (loué, acheté ou consigné).
* Le coût des recharges de gaz.
* Le coût des détendeurs et/ou manodétendeurs indispensables.
* Les contraintes d'approvisionnement, de stockage de l'hydrogène et les dangers que cela représente.
* En général, toutes difficultés liées à l'emploi de l'hydrogène.
- Un deuxième procédé consiste à soumettre un courant gazeux chargé de vapeurs d'essence de térébenthine à une insolation de rayons ultra-violets. Mais ce procédé donne des résultats insuffisants quand aux produits obtenus et à leurs applications médicales particulièrement en inhalations, ce, compte-tenu de la nocivité qu'il y a à inhaler des vapeurs d'essence de térébenthine.

En effet, ce deuxième procédé est, en son état actuel, incapable de transformer correctement tout un courant gazeux chargé de vapeurs d'essence de térébenthine en péroxydes de terpène oxoniums, faisant que subsistent encore très facheusement en fin du dit procédé, et en proportion trop importante, des vapeurs d'essence de térébenthine non transformées.

- Un troisième procédé consiste à envoyer un courant gazeux chargé de vapeurs d'essence de térébenthine au travers d'un arc électrique. Mais ce procédé donne des résultats insuffisants quand aux produits obtenus et à leurs applications médicales particulièrement en inhalations, ce, compte-tenu de la nocivité qu'il y a à inhaler des vapeurs d'essence de térébenthine, de l'ozone et des dérivés ozoniques.

En effet, ce troisième procédé, en son état actuel, outre qu'il soit, pareillement au deuxième procédé, incapable de transformer correctement tout un courant gazeux chargé de vapeurs d'essence de térébenthine en péroxydes de terpène oxoniums, produit de plus, et de façon à ce que subsiste encore très facheusement en fin du dit procédé, et en proportion trop importante, de l'ozone et des dévivés ozoniques.

L'invention consiste en un nouveau procédé qui, à partir des procédés "arc électrique" et, ou "rayons ultra-violets" ci-dessus résumés, permet de transformer correctement tout un courant gazeux chargé de vapeurs d'essence de térébenthine en péroxydes de terpène oxoniums, ce, en quantité massive, immédiatement disponible et à un niveau optimal de pureté, sans qu'il y subsiste alors (ou en quantité infinitésimale), ni vapeur d'essence de thérébenthine non transformée, ni ozone, ni dérivés ozoniques indésirables.

L'invention permettant d'obtenir les produits (péroxydes de terpène oxoniums) et les résultats ci-dessus décrits, consiste à envoyer un courant gazeux chargé de vapeurs d'essence de térébenthine vers un quelconque dispositif (A) placé dans l'environnement proche (B) de l'arc électrique, et, ou du générateur de rayons ultra-violets, pour refroidir ce dispositif, initialement porté à une température comprise entre 55°C et 2.950°C, jusqu' à une température comprise entre 35°C et 280°C.

D'après l'invention, l'un ou l'autre des deux procédés : "arc électrique" et, ou "rayons ultra-violets" peut être utilisé, et, ou encore très avantageusement la combinaison de ces deux procédés, combinaison favorisée par l'utilisation d'arcs électriques appropriés, producteurs de rayonnements ultra-violets.

Les moyens et, ou les conditions d'une telle invention peuvent être facilement obtenus, et nous en citons ici quelques exemples.

La figure 1 représente un premier exemple ilustrant ce procédé et le dispositif d'un tel procédé.

La figure 2 représente un deuxième exemple ilustrant ce procédé et le dispositif d'un tel procédé.

La figure 3 représente un troisième exemple ilustrant ce procédé et le dispositif d'un tel procédé.

## 1° EXEMPLE :

Certains arcs électriques (et l'on se méfiera ici des tranferts de matière, pouvant être responsable d'une production de dérivés indésirables) sont générateur de chaleur, et à ce titre donc, peuvent être considérés comme étant l'un des quelquonques moyens décits dans la présente invention, et, ou, encore, un tube en métal, par exemple formant chambre et recevant la chaleur de cet arc électrique, tube dans lequel circulera le courant gazeux.

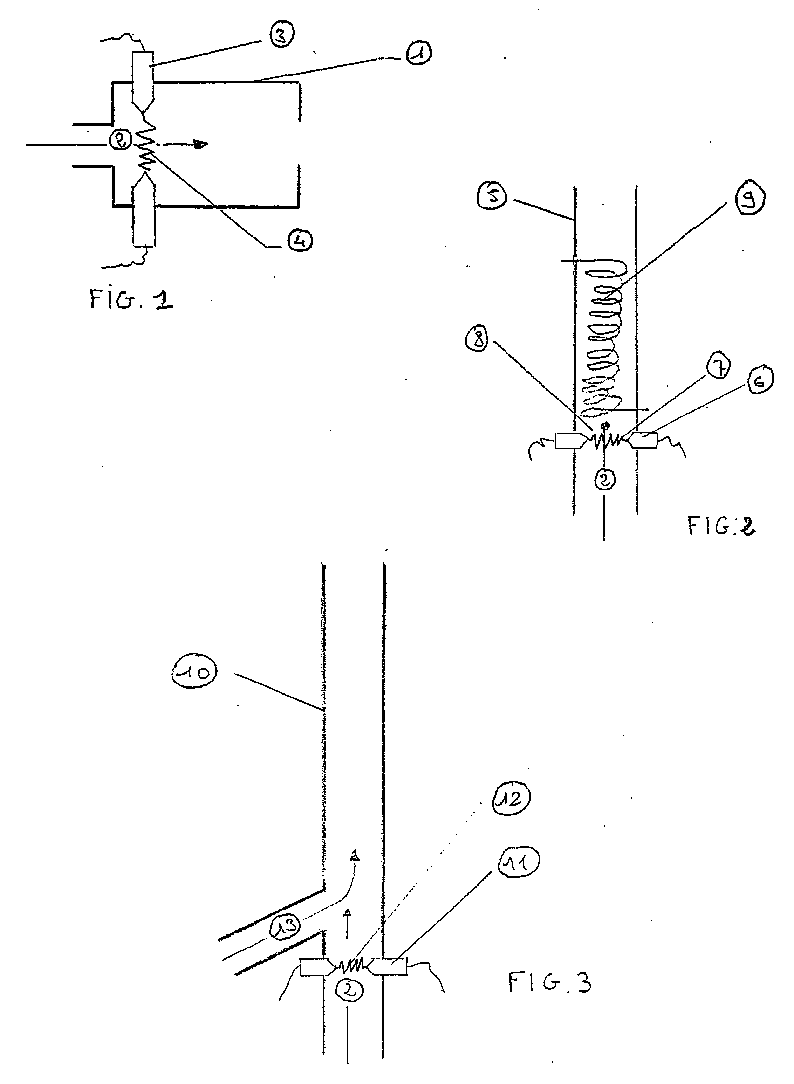

Ainsi, reprenant ces explications, dans un tube (1) par où arrive un courant gazeux chargé de vapeurs d'essence de térébenthine (2) seront fichées des électrodes (3) produisant un arc électrique chaud (4), le tube, et, ou l'arc électrique initialement porté(s) à bonne température étant refroidi-(s) par le courant gazeux, ce dans les conditions de température décrites dans la présente invention : FIGURE 1.

## 2° EXEMPLE :

Certains arcs électriques sont peu générateurs de chaleur, notament ceux d'origine électronique, souvent aussi grands générateurs de rayonnement ultra-violets, et généralement peu enclin à des transferts de matières.

Ces arcs électriques, contrairement à l'exemple 1 ne pourraient être considéré comme étant l'un des quelquonques dispositifs décrits dans la présente invention. Celui-ci pourrait alors être :
- Une résistance électrique judicieusement placé et correctement refroidie après avoir été ammenée à bonne température.
- Un courant d'air chaud judicieusement amené et correctement refroidi après avoir été porté à bonne température.
- Et en général, tout autre quelquonque dispositif correctement refroidi après avoir été porté à bonne température.

Ainsi, reprenant ces explications, dans un tube (5) par où arrive un courant gazeux chargé de vapeurs d'essence de térébenthine (2) seront fichées des électrodes (6) produisant un arc électrique (7) générateur de rayons ultra-violets (8), et aussi une résistance électrique (9). la résistance électrique et, ou éventuellement le tube, étant refroidi(es) par le courant gazeux après avoir été porté(es) à bonne température, ce dans les conditions de température décrites dans la présente invention : FIGURE 2.

Ainsi, reprenant les explications ci-dessus, dans un tube (10) où arrive un courant gazeux chargé de vapeur d'essence de térébenthine (2) seront fichées des électrodes (11) produisant un arc électrique (12), et dans ce tube (10) sera aussi amené un courant d'air chaud (13), ce courant d'air chaud, et, ou éventuellement le tube, étant refroidi-(s) par la courant gazeux, ce dans les conditions de température décrites dans la présente invention : FIGURE 3.

Les mêmes exemples et dispositifs sont aussi valable, s'agissant non plus d'un arc électrique (ou de plusieurs arcs électriques), mais d'un générateur quelquonque produisant un rayonnement d'ultra-violets.

Il suffira donc de remplacer l'arc électrique par un générateur de rayons ultra-violets.

Les mêmes exemples et dispositifs sont aussi valable, s'agissant non plus d'un arc électrique (ou de plusieurs arcs électriques), ou d'un générateur quelquonque produisant un rayonnement d'ultra-violets, mais de la combinaison de ces deux moyens.

Il suffira donc de remplacer l'arc électrique ou un générateur de rayons ultra-violets par la combinaison de ces deux moyens.

Ce nouveau procédé et en ses dispositifs favorisent très grandement et intensivement les oxydations du fait du rayonnement des rayons ultra-violets, et, ou de l'ozone et, ou des dérivés ozoniques produits par l'arc électrique (ou plusieurs arcs électriques) permettant selon l'invention, d'obtenir des péroxydes de terpène oxoniums, ce, en quantité massive, immédiatement disponible et à un niveau optimal de pureté.

L'on comprendra aisément l'intéret industriel et commercial d'une telle invention, ainsi :

Il pourra être fabriqué des appareils produisants des péroxydes de terpène oxoniums ce, en quantité massive, immédiatement disponible et à un niveau optimal de pureté, les exemples ci-dessus indiquants, entre autres, de quelles façons.

La commercialisation sera grandement facilité par rapport aux appareils utilisants l'hydrogène, grace au fait que précisément, ces nouveaux appareils n'utilisent plus l'hydrogène avec son cortège d'inconvéniants.

Le marché locatif devient facilement abordable, et très prometteur.

ANNEXE

(A) DISPOSITIF : Pris au sens large du terme, indiquant en général toute(s) chose(s) pouvant être chauffée(s) par n'importe(s) quel(s) moyen(s) de chauffage.

(B) de préférence, en considérant le sens de progression du courant gazeux chargé de vapeurs d'essence de térébenthine, en amont et très proche de l'arc électrique et, ou du générateur de rayons ultra-violets.

**Revendications**

1°) Procédé pour la production de péroxydes de terpène oxoniums caractérisé par le fait qu'un dispositif initialement porté à une température comprise entre 55° C et 2.950° C soit refroidit par un courant gazeux chargé de vapeurs d'essence de térébenthine, jusqu'à une température comprise entre 35° C et 380° C.

2°) Procédé selon la revendication 1 caractérisé par le fait que le dispositif soit placé dans l'environnement proche d'un arc électrique (et, ou des arcs électriques), et, ou d'un rayonnement d'utra-violets, et, ou encore, très avantageusement, dans l'environnement de la combinaison de ces deux moyens. Environnement (voir B)

3°) Procédé selon la revendication 1 et 2 caractérisé par le fait que le courant gazeux chargé de vapeurs d'essence de térébenthine soit soumis à l'action de l'arc électrique (et, ou des arcs électriques), et, ou du rayonnement ultra-violets, tout en refroidisant le dispositif initialement porté à une température comprise entre 55° C et 2.950° C, jusqu'à une température comprise entre 35° C et 380° C.

4°) Procédé selon l'une quelquonque des revendications précédentes caractérisé par le fait que le dispositif de ce procédé puisse être précisément un arc électrique (ou plusieurs arcs électriques), (4) combien même celui-ci (ou ceux-ci) serait déjà rattaché à un autre procédé.

5°) Procédé selon l'une quelquonque des revendications précédentes caractérisé par le fait que le dispositif de ce procédé puisse être aussi un tube (1) sur lequel sont fichées des électrodes.

6°) Procédé selon l'une quelquonque des revendications précédentes caractérisé par le fait que le dispositif de ce procédé puisse être aussi des électrodes (3) produisant un arc électrique.

7°) Procédé selon l'une quelquonque des revendications précédentes caractérisé par le fait que le dispositif de ce procédé puisse être une résistance électrique (9), et éventuellement le tube (5) dans lequel elle est fichée.

8°) Procédé selon l'une quelquonque des revendications précédentes caractérisé par le fait que le dispositif de ce procédé puisse être un courant d'air chauffé (13), et éventuellement le tube (10) dans lequel il est amené.

9°) Procédé selon l'une quelquonque des revendications précédentes caractérisé par le fait que le dispositif de ce procédé puisse être toutes autre(s) chose(s), pouvant être initialement chauffée(s), puis refroidite(s) par un courant gazeux chargé de vapeur d'essences de térébenthine, ce dans les conditions de température décrites dans la présente invention.

FIG. 1

FIG. 2

FIG. 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| E | FR-A-2 609 028 (P. GUICHARD) <br> * En entier * <br> --- | 1-9 | C 07 C 179/06 <br> C 07 C 178/00 |
| D,A | GB-A- 651 965 (R.L. JACQUIER) <br> * En entier * <br> ----- | 1 | |

DOMAINES TECHNIQUES
RECHERCHES (Int. Cl.4)

C 07 C 179/00
C 07 C 178/00

Le présent rapport a été établi pour toutes les revendications

| Lieu de la recherche | Date d'achèvement de la recherche | Examinateur |
|---|---|---|
| LA HAYE | 16-02-1989 | BONNEVALLE E.I.H. |